# EUROPEAN PATENT SPECIFICATION

(11) **EP 3 877 047 B1**
(45) Date of publication and mention of the grant of the patent: **19.04.2023**
(21) Application number: 20737991.8
(22) Date of filing: 06.01.2020
(51) Int. Cl.: A61N 5/06

(54) **PHOTOTHERAPY DEVICE FOR IMPROVING SEMEN QUALITY**
FOTOTHERAPIEVORRICHTUNG ZUR VERBESSERUNG DER SAMENQUALITÄT
DISPOSITIF DE PHOTOTHÉRAPIE POUR AMÉLIORER LA QUALITÉ DU SPERME

(30) Priority: 08.01.2019 LV 190003
(43) Date of publication of application: 15.09.2021
(73) Proprietor: Ferterex, Sia, 1001 Riga (LV)
(72) Inventor: BELTE, Maris, 2011 Jurmala (LV)
(74) Representative: Fortuna, Jevgenijs
(86) International application number: PCT/LV2020/050001
(87) International publication number: WO 2020/145809

(56) References cited:
- WO-A1-02/05895
- KR-B1- 100 889 296
- KR-B1- 100 889 296
- US-A1- 2014 303 693
- US-A1- 2015 141 881
- US-A1- 2016 166 833

## Description

### Technical Field

The invention relates to phototherapy devices, more particularly - phototherapy devices adapted for improving human semen quality.

### Background Art

Semen quality is a measure of the ability of semen to accomplish fertilisation. Spermatozoa having insufficient or unsuitable motility do not reach an ovule. This is one of the main causes of subfertility or infertility.

Phototherapy with low level light is widely used in the treatment of various illnesses. Photostimulation affects enzymes in the cellular respiratory chain, contributes to the biosynthesis of ATP and signalling substances. Both *in vitro* and *in vivo* studies have shown that phototherapy transcutaneously applied to semen or testicles can improve sperm parameters and increase testosterone level (Ban Fragez H et al.; Photobiomodulation with light-emitting diodes improves sperm motility in men with asthenozoospermia. Lasers Med Sci. 2015 Jan;30(1):235-40. doi: 10.1007/s10103-014-1653-x. Epub 2014 Sep 10. Moskvin S. V. et al.; Effectiveness of low level laser therapy for treating male infertility. Biomedicine (Taipei). 2018 Jun;8(2):7 doi:10.1051/bmdcn/2018080207. Epub 2018 May 28).

There is known a photo-therapy device (US6896693) comprising an enclosure having a hollow shaped bottom and a movable lid attached to the bottom, an array of LEDs arranged in the lid, an array of LED arranged in the bottom, the arrays of LEDs are designed to emit infrared light, a control circuit housed within the hollow shaped bottom or in the lid, the control circuit being electrically connected to the arrays of LEDs for controlling the operation of the arrays of LEDs.

KR 100889296 B1 discloses male-hormone accelerating and sperm activating device, comprising a housing provided with a cavity designed for receiving human testicles, the housing provided with temperature sensor, cooling fan, temperature control unit, light emitting diode, low power laser diode, input unit and control unit. The cavity is provided with light emitting diode and low power laser diode designed to emit light towards human's testicles placed in said cavity.

Major drawbacks of the known solutions is their relative insufficient effectiveness, use limitations and relatively high production costs.

### Disclosure of the Invention

The device according to the invention comprises a housing, a plurality of light emitting diodes (LED); a switch for operating the light emitting diodes; an electrical circuit having a controller encased in the housing, the circuit being electrically connected with the switch, the power source and the LEDs for delivering power and operational control from the power source to the LEDs upon activation by the switch, the controller programmed with at least one light pattern selected and displayed on the plurality of LEDs during operation of the device.

The housing having two ends: one and two, where end one has a cavity adapted to receive human's testicles and end two is designed to be located under male's crotch and part of buttocks adjacent to crotch. Thus, the end two of the housing is designed to conform to the shape of a seated thereon person's crotch. The cavity in the end one has two testicle conforming areas capable of receiving and separating male testicles. This design of the housing allows to securely fix the device on a flat surface by the weight of a human sitting on the device in operational position.

The plurality of light emitting diodes are positioned in the cavity in the end one of the housing, wherein at least one light emitting diode is secured to the surface of the upper part of the end two of the housing, so that the light emitting diode in the end two of the housing is positioned substantially under prostate of a person seated on the device. The light emitting diodes in the cavity in the end one of the housing are spaced apart at discrete intervals along the length of the cavity relative to other light emitting diodes.

### Brief Description of Drawings

Fig. 1 shows perspective view of one embodiment of the phototherapy device for improving semen quality;
Fig. 2 shows longitudinal section of the phototherapy device;
Fig 3A-3C show three embodiments of the phototherapy device with different positioning of light emitting diodes in the end one and end two of the device housing;
Fig. 4A and 4B show the device in operational position with male sited thereon; where

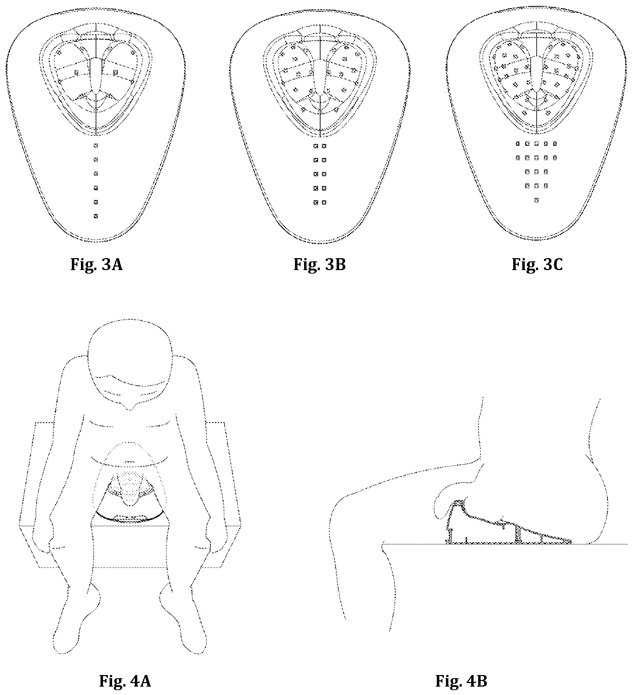

Fig. 4A shows schematic view from the top and Fig. 4B - shows schematic longitudinal section of the side view;

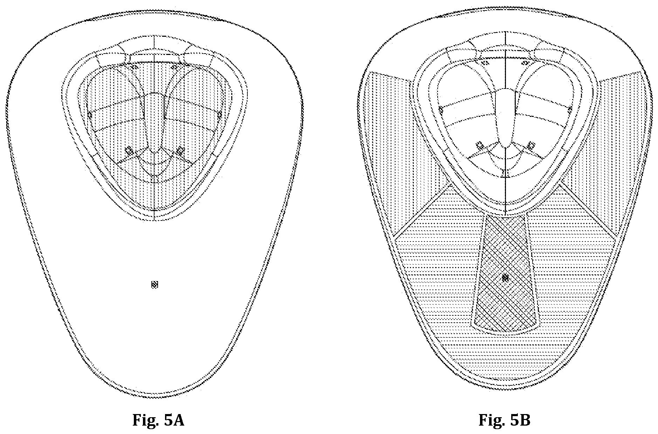

Fig. 5 shows top view of the device in operation with indication of areas of the housing, where sited user's body parts are in contact with the housing; wherein Fig. 5A shows cavity in the end one of the housing, which is in contact with male user's testicles and Fig. 5B - areas of the device, which are in contact with user's crotch, buttocks and legs.

### Detailed Description of the Invention

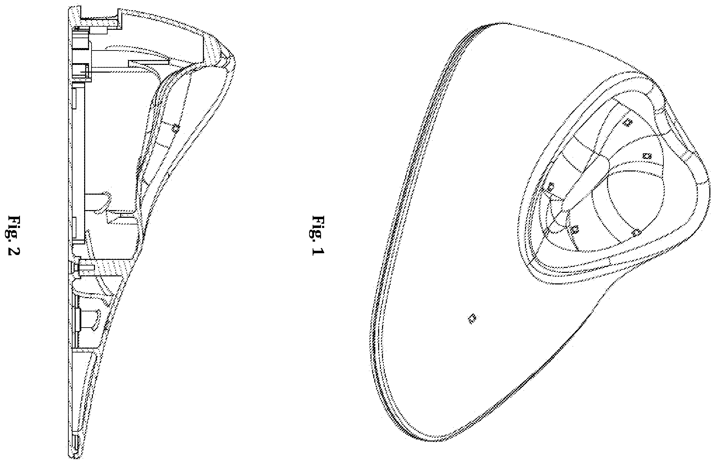

The phototherapy device (Fig. 1, 2) comprises a housing (1), a plurality of light emitting diodes (2); a switch (3) for operating the light emitting diodes (2); optionally - a power source (e.g. regular battery or rechargeable battery encased in the housing (1) or external power source) for powering the light emitting diodes (2); an electrical circuit having a controller encased in the housing (1), the circuit being electrically connected with the switch (3), the power source and the LEDs (2) for delivering power and operational control from the power source to the LEDs (2) upon activation by the switch (3), the controller programmed with at least one light pattern selected and displayed on the plurality of LEDs (2) during operation of the device.

According to the preferred embodiment the switch (3) is positioned on the housing (1). The battery is encased in the housing (1). The controller may be programmed with a plurality of light patterns, which can be selected and displayed on the plurality of LEDs (2) during operation of the device.

The housing (1) having two ends: one and two, where end one has a cavity (4) adapted to receive human's testicles and end two - a protrusion (5), designed to be located under male's crotch and, optionally, - part of buttocks adjacent to crotch. Thus, the end two of the housing is designed to conform to the shape of a seated thereon person's crotch. This design of the housing (1) allows to securely fix the device on a flat surface by the weight of a human sitting on the device in operational position. The cavity (4) in the end one has two testicle conforming areas (4' and 4") capable of receiving and separating male testicles. Each testicle conforming areas (4' and 4") comprising a raised peripheral area (6), whereby the central area (7) between the testicle conforming areas (4' and 4") curves upward to support separation of male testicles.

The plurality of the light emitting diodes (2) are positioned in the cavity (4) in the end one of the housing. The LEDs (2) in the cavity (4) in the end one of the housing (1) are spaced apart at discrete intervals along the length of the cavity (4) relative to other LEDs (2). According to one embodiment, the LEDs (2) in the cavity (4) are located on the raised peripheral areas (6) and on the lower portions of the testicle conforming areas (4' and 4"). Optionally, the LEDs (2) may be also located on the central area (7) between the testicle conforming areas (4' and 4"), so that the light is emitted from three different sides of user's testicles.

At least one light emitting diode (2) is secured to the surface of the protrusion (5), so that the LED (2) in the end two of the housing (1) is positioned substantially under prostate of a person seated on the device (Fig. 1). However, the number of light emitting diodes (2) on the protrusion (5) on the end two of the housing (1) can be substantially bigger than one. Also, as shown on Fig. 3A, 3B and 3C, the light emitting diodes (2) may cover larger area substantially under a seated person prostate and close to it. As shown on Fig. 3A and 3B the light emitting diodes (2) may be centrally located on the protrusion (5) on the end two of the housing (1) in one or more than one lines, thereby ensuring more effective irradiation of prostate by low level light.

According to the preferred embodiment the LEDs (2) are positioned in the housing (1) so that they bulge outwards for 0,5 - 3 mm. This ensures low pressure on the skin, resulting in blood being pushed out of the subcutaneous capillary network, and light energy is better penetrated to target tissues. This way a better effect can be achieved with less heat being released.

According to the invention the LEDs (2) having wavelength from 650 to 1080 nM, power from 5 to 100 mW and beam angle from 20 to 120° are selected. The device is configured to provide from 60 to 360 J energy to testicles with energy density from 2 to 12 J/cm².

The controller is pre-programmed for delivering power from the power source to the LEDs (2) for about 3 to 15 minutes.

According to one embodiment the housing (1) is made of a rigid material. According to another embodiment the housing (1) is made of a deformable material.

The device operates as follows. After activation of the switch (3) the selected light pattern is displayed on the plurality of LEDs (2). As shown on Fig. 4A and 4B, a male user sits on the device, so that his testicles are placed in the cavity (4), but his crotch - on the protrusion (5). Fig. 5A shows the area of the housing (1), namely, the cavity (4), where sited user's testicles are in contact with the housing. Fig. 5B shows the areas of the device, which are in contact with user's crotch (this area is shown on Fig. 5B by crosshatching), buttocks (this area is shown on Fig. 5B by horizontal hatching) and legs (this area is shown on Fig. 5B by vertical hatching). It should be noted that Fig. 5B is one possible embodiment and that other confuguration of the housing according to the invention is possible.

Regular use of the proposed device allows to increase number of spermatozoa, their motility, increase testosterone level and decrease FSH levels.

## Claims

1. A phototherapy device for improving semen quality, comprising a housing (1), a plurality of light emitting diodes (2), a switch (3), an electrical circuit having a controller, the circuit being electrically connected with the switch (3), power source and the light emitting diodes (2) for delivering power and operational control from the power source to the light emitting diodes (2) upon activation by the switch (3), the controller programmed with at least one light pattern selected and displayed on the plurality of the light emitting diodes (2) during operation of the device, **characterized in that** the housing (1) has two ends: one and two, where end one has a cavity (4) with two testicle conforming areas (4' and 4") capable of receiving and separating male testicles; end two has a protrusion (5), designed to conform to the shape of a seated thereon person's crotch and, optionally - also part of buttocks; wherein the plurality of light emitting diodes (2), spaced apart at discrete intervals, are positioned in the cavity (4) in the end one of the housing (1) so that they bulge outwards for 0.5 to 3 mm, where at least one light emitting diode (2) is secured to the surface of the end two of the housing (1), so that the light emitting diode (2) in the end two of the housing (1) is positioned substantially under prostate of a person seated on the device.

2. The phototherapy device according to claim 1, wherein the light emitting diodes (2) are LEDs having wavelength from 650 to 1080 nm, power from 5 to 100 mW and beam angle from 20 to 120°, providing from 60 to 360 J energy to testicles with energy density from 2 to 12 J/cm².

3. The phototherapy device according to claim 1 or 2, wherein the plurality of light emitting diodes (2), spaced apart at discrete intervals are secured to the upper surface of the housing's (1) end two portion.

4. The phototherapy device according to claim 3, wherein the light emitting diodes (2) are centrally located on the protrusion (5) on the end two of the housing (1) in one or more lines.

5. The phototherapy device according to any preceding claims, wherein the cavity (4) comprises a central area (7) between the testicle conforming areas (4' and 4"), whereby the central area (7) curves upward to support separation of male testicles.

6. The phototherapy device according to any preceding claims, wherein the housing (1) is made of a rigid material.

7. The phototherapy device according to any preceding claims, wherein the housing (1) is made of a deformable material.

## Patentansprüche

1. Phototherapiegerät zur Verbesserung der Samenqualität, bestehend aus einem Gehäuse (1), einer Vielzahl von Leuchtdioden (2), einem Schalter (3), einem elektrischen Schaltkreis mit Steuerung, wobei der Schaltkreis elektrisch mit dem Schalter (3) verbunden ist, einer Stromquelle und Leuchtdioden (2), um die Leuchtdioden (2) bei Aktivierung durch den Schalter (3) mit Strom und Betriebssteuerung von der Stromquelle aus zu versorgen, wobei die Steuerung mit mindestens einem Lichtmuster, das ausgewählt und auf der Vielzahl der Leuchtdioden (2) während des Gerätebetriebs angezeigt wird, programmiert ist. Das Phototherapiegerät **ist dadurch gekennzeichnet, dass** das Gehäuse (1) zwei Enden aufweist: eins und zwei, wobei Ende eins einen Hohlraum (4) mit zwei sich an die Hoden anpassenden Stellen (4' und 4") aufweist, die in der Lage sind, männliche Hoden aufzunehmen und voneinander zu trennen; Ende zwei einen Vorsprung (5) aufweist, der so gestaltet ist, dass er sich an die Form des Schritts einer darauf sitzenden Person und - optional - auch an einen Teil des Gesäßes anpasst. Die Mehrzahl der Leuchtdioden (2), die sich in diskreten Abständen voneinander getrennt befinden, ist in dem Hohlraum (4) an Ende eins des Gehäuses (1) positioniert, so dass sie um 0,5 bis 3 mm nach außen hin gewölbt angeordnet sind, wobei mindestens eine Leuchtdiode (2) an der Oberfläche von Ende zwei des Gehäuses (1) befestigt ist, so dass die Leuchtdiode (2) an Ende zwei des Gehäuses (1) im Wesentlichen unter der Prostata einer auf der Vorrichtung sitzenden Person positioniert ist.

2. Phototherapiegerät gemäß Patentanspruch 1, wobei es sich bei den Leuchtdioden (2) um LEDs mit einer Wellenlänge von 650 bis 1080 nm, einer Leistung von 5 bis 100 mW und einem Abstrahlwinkel von 20 bis 120° handelt, die den Hoden eine Energie von 60 bis 360 J mit einer Energiedichte von 2 bis 12 J/cm² zuführen.

3. Phototherapiegerät gemäß Patentanspruch 1 oder 2, wobei die mehreren Leuchtdioden (2), die in diskreten Abständen voneinander angeordnet sind, an der oberen Fläche des Gehäuseabschnitts von Ende zwei (1) befestigt sind.

4. Phototherapiegerät gemäß Patentanspruch 3, wobei die Leuchtdioden (2) mittig auf dem Vorsprung (5) von Ende zwei des Gehäuses (1) in einer oder mehreren Reihen angeordnet sind.

5. Phototherapiegerät gemäß einem der vorhergehenden Patentansprüche, wobei der Hohlraum (4) einen zentralen Bereich (7) zwischen sich an die Hoden anpassenden Stellen (4' und 4") aufweist. Der zentrale Bereich (7) ist dabei nach oben gekrümmt ist, um die Trennung der männlichen Hoden voneinander zu unterstützen.

6. Phototherapiegerät gemäß einem der vorhergehenden Patentansprüche, wobei das Gehäuse (1) aus einem starren Material besteht.

7. Phototherapiegerät gemäß einem der vorhergehenden Patentansprüche, wobei das Gehäuse (1) aus einem verformbaren Material hergestellt ist.

## Revendications

1. Dispositif de photothérapie pour améliorer la qualité du sperme, comprenant un logement (1), une pluralité de diodes électroluminescentes (2), un interrupteur (3), un circuit électrique ayant un dispositif de commande, le circuit étant électriquement connecté à l'interrupteur (3), à une source de puissance et aux diodes électroluminescentes (2) pour délivrer de la puissance et une commande fonctionnelle depuis la source de puissance vers les diodes électroluminescentes (2) lors de l'activation par l'interrupteur (3), le dispositif de commande programmé avec au moins un motif lumineux sélectionné et affiché sur la pluralité des diodes électroluminescentes (2) pendant le fonctionnement du dispositif, **caractérisé en ce que** le logement (1) a deux extrémités : une et deux, où l'extrémité une a une cavité (4) avec deux zones (4" et 4") s'adaptant à la forme de testicules, capables de recevoir et de séparer des testicules mâles ; l'extrémité deux a une partie saillante (5), destinée à s'adapter à la forme d'un entrecuisse d'une personne assise sur celle-ci et, facultativement - également une partie des fesses ; dans lequel la pluralité de diodes électroluminescentes (2), espacées selon des intervalles distincts, sont positionnées dans la cavité (4) dans l'extrémité une du logement (1) de sorte qu'elles font saillie vers l'extérieur sur 0,5 à 3 mm, où au moins une diode électroluminescente (2) est fixée à la surface de l'extrémité deux du logement (1), de sorte que la diode électroluminescente (2) dans l'extrémité deux du logement (1) est positionnée sensiblement sous la prostate d'une personne assise sur le dispositif.

2. Dispositif de photothérapie selon la revendication 1, dans lequel les diodes électroluminescentes (2) sont des DEL ayant une longueur d'onde allant de 650 à 1080 nm, une puissance de 5 à 100 mW et un angle de faisceau allant de 20 à 120°, fournissant de 60 à 360 J d'énergie à des testicules avec une densité énergétique allant de 2 à 12 J/cm².

3. Dispositif de photothérapie selon la revendication 1 ou 2, dans lequel la pluralité de diodes électroluminescentes (2), espacées à des intervalles distincts sont fixées à la surface supérieure de la partie d'extrémité deux du logement (1).

4. Dispositif de photothérapie selon la revendication 3, dans lequel les diodes électroluminescentes (2) sont situées en position centrale sur la partie saillante (5) sur l'extrémité deux du logement (1) dans une ou plusieurs lignes.

5. Dispositif de photothérapie selon de quelconques revendications précédentes, dans lequel la cavité (4) comprend une zone centrale (7) entre les zones (4" et 4") s'adaptant à la forme de testicules, moyennant quoi la zone centrale (7) s'incurve vers le haut pour supporter une séparation de testicules mâles.

6. Dispositif de photothérapie selon de quelconques revendications précédentes, dans lequel le logement (1) est réalisé en un matériau rigide.

7. Dispositif de photothérapie selon de quelconques revendications précédentes, dans lequel le logement (1) est réalisé en un matériau déformable.
